# EUROPEAN PATENT APPLICATION

(11) **EP 4 592 680 A1**
(43) Date of publication of application: **30.07.2025**
(21) Application number: 24382061.0
(22) Date of filing: 24.01.2024
(51) Int. Cl.: G01N 33/574

(54) **PROGNOSTIC BIOMARKERS FOR DETECTION OF COLORECTAL CANCER**

(71) Applicant: Fundació Institut d'Investigació Sanitària Pere Virgili (IISPV), 43003 Tarragona (ES); Universitat Rovira I Virgili (URV), 43003 Tarragona (ES); Biosfer Teslab, S.L., 43204 Reus (ES)
(72) Inventor: CUMERAS OLMEDA, Raquel, 43003 Tarragona (ES); GUMÀ PADRÓ, Josep, 43003 Tarragona (ES); MIRACLE HUGUET, Cristina, 43003 Tarragona (ES); AMIGÓ GRAU, Núria, 43204 Reus (ES); OZCARIZ GARCÍA, Enrique, 43204 Reus (ES)
(74) Representative: Hoffmann Eitle

(57) **Abstract**

The present invention relates to a method for detecting from a urine sample whether a subject has or is predisposed to developing metastatic colorectal cancer, comprising obtaining a metabolite profile from said urine sample by measuring the concentration of the metabolite trimethylamine N-oxide (TMAO) alone and/or in combination with the metabolite cis-aconitate and/or the metabolite valine.

## Description

### TECHNICAL FIELD

The present invention relates to a method for detecting from a urine sample whether a subject has or is predisposed to developing metastatic colorectal cancer, comprising obtaining a metabolite profile from said urine sample by measuring the concentration of the metabolite trimethylamine N-oxide (TMAO) alone and/or in combination with the metabolite cis-aconitate and/or the metabolite valine.

### BACKGROUND OF THE INVENTION

Colorectal cancer (CRC) causes the second most global cancer deaths, after lung cancer (1). Often, CRC does not present symptoms until later and more dangerous stages of the disease, which makes screening important for reducing mortality. In resectable CRCs, post-resection surveillance is vital to reduce the mortality associated with cancer recurrence, as 30-50% of patients currently experience fatal relapse of their cancers (2). State-of-the-art surveillance methods are time and resource-intensive, involving frequent colonoscopies, imaging, and blood testing, and the current serum-based biomarkers like CEA have suboptimal diagnostic utility for recurrence (3), as they have very low sensitivity (47%) but good specificity (80%) (4). In addition, CEA tests rely on blood requiring invasive treatment every time a diagnostic is to be performed.

Furthermore, current recommended imaging techniques for the diagnosis of metastases are imperfect. The current imaging techniques recommended for detecting metastases, such as computed tomography (CT), magnetic resonance imaging (MRI), and positron emission tomography (PET), are not always able to identify small lesions or micro metastases that may be present in the body (5).

There are two major problems in the management of colon cancer that are still not solved by the detection methods of the prior art. One is the discrimination of metastatic CRC from early CRC during initial diagnosis, and the other is the detection of CRC recurrence during postoperative surveillance. There is therefore a need for the development of a diagnostic method and device that increases the overall sensitivity, specificity, non-invasiveness and cost-effectiveness of the diagnosis of mCRC. Ultimately such a method and device can reduce unnecessary surgeries or biopsies in early, non-metastatic cases and earlier intervention in metastatic disease which will improve patient outcomes and lower the economic costs of treatment.

With the current prior art detection systems highly reliable results are obtained when urine samples are processed, e.g., by NMR spectroscopy, in a laboratory setting. However, in many instances, it may be desirable to provide immediate results in a clinician's office or to permit a subject to even conduct testing at home. The need for a test that is portable, prepackaged, disposable, usable by a subject without assistance or direction, etc. may in some instances be of more importance than a high degree of accuracy.

The inventors have therefore set out to develop a method as well as a simple and cost-efficient device to put the method in practice that will overcome the above disadvantages.

Various metabolites have been suggested in the prior art for detection of CRC, mainly in serum samples. In one study a total of 249 different metabolites have been identified in the serum of CRC patients from which 72 metabolites were found differentially expressed in CRC subjects showing that serum-based metabolomics is able to discriminate CRC patients from healthy controls (6). However, the attempt to stratify TNM stages (I-IV) of CRC patients using these differential metabolites was not successful.

Trimethylamine N-oxide (TMAO), one of the 72 metabolites identified in that study has been described as a potentially important intermediate marker linking dietary meat and fat and gut microbiota metabolism to risk of CRC (7). Liu X. et al (2017) suggested pre-treatment serum TMAO level as a new independent prognostic biomarker in patients with colorectal cancer, especially in stage II and/or III patients, however, acknowledging that further studies with large sample size and better controlled subjects are needed to confirm the value of TMAO as a new prognostic marker for colorectal cancer patients (8). Stage IV CRC patients having already developed metastatic CRC were not assessed.

In a urinary metabolomic study on a cohort of CRC subjects a panel of 35 metabolite markers including TMAO were selected and TMAO was found depleted in the urine of CRC subjects (9). Finally, from the panel of 35, the following seven representative metabolites in urine were selected as a panel of candidate urine markers to discriminate CRC subjects from their healthy counterparts: citrate, hippurate, p-cresol, 2-aminobutyrate, myristate, putrescine, and kynurenate. TMAO was not among these markers.

Whilst a link between TMAO and colorectal cancer has been previously described, the specific use of TMAO as a metabolic marker in urine for detection of metastatic colorectal cancer, or for the discrimination of the stages of said cancer has not been suggested.

Methods for the early diagnosis of CRC by measurement of more than one metabolite in a sample, such as for example blood, saliva, serum or urine, are described for example in WO2011/143779 and US2012/0040383. In both cases TMAO is listed among a large number of possible metabolites, but not selected as suitable a metabolic marker.

Another known metabolite, cis-aconitate, is also listed among the many metabolic markers disclosed in the method of CRC diagnosis in WO2011/143779, however, it is neither alone nor in combination with TMAO described as a specifically advantageous choice of metabolic marker for the detection of CRC.

### SUMMARY OF THE INVENTION

The present invention therefore relates in one aspect to a method for detecting whether a subject has or is predisposed to developing metastatic colorectal cancer, said method comprising:
(a) providing a urine sample from said subject;
(b) obtaining a metabolite profile from said urine sample, wherein said metabolic profile is obtained by measuring the concentration of at least the metabolite TMAO in said urine sample to produce said metabolite profile for said subject;
(c) comparing said metabolite profile with a reference metabolite profile, wherein said reference metabolite profile is determined from the concentration of corresponding metabolites in urine of individuals in a reference population; and
(d) assessing, based on said comparison in step (c), whether said subject has or is predisposed to developing metastatic colorectal cancer.

In one embodiment of the method of present invention an elevated urine concentration of TMAO is indicative of that the subject has or is predisposed to developing metastatic colorectal cancer.

In one embodiment said elevated urine concentration of TMAO is a urine concentration of more than 9 mmol/mL, preferably more than 9.5 mmol/mL, more preferably more than 10 mmol/mL, most preferred more than 10.5 mmol/mL.

In one embodiment, said elevated urine concentration can be up to 35 mmol/mL.

In one embodiment, said elevated urine concentration is between 9 mmol/mL to 35 mmol/mL.

In one embodiment of the method of present invention step b) furthermore comprises measuring the concentration of the metabolite cis-aconitate in said urine sample.

In one embodiment of the method of present invention the reduced urine concentration of cis-aconitate is indicative of that the subject has or is predisposed to developing metastatic colorectal cancer.

In one embodiment said reduced urine concentration of cis-aconitate is a urine concentration of less than 4 mmol/mL, preferably less than 3.5 mmol/mL, more preferably less than 3 mmol/mL, or less than 2.5 mmol/mL, most preferred less than 2.0 mmol/mL.

In one embodiment the urine concentration of cis-aconitate is between 4 mmol/mL and 1.3 mm/mL, 3.5 mmol/mL and 1.3 mm/mL, between 3 mmol/mL and 1.3 mm/mL, between 2.5 mmol/mL and 1.3 mm/mL, most preferred between 2.0 mmol/mL and 1.3 mm/mL.

In one embodiment said metabolic profile is obtained by further measuring the concentration of at least one other metabolite, wherein said other metabolite is valine.

In one embodiment the elevated urine concentration of valine is indicative of that the subject has metastatic colorectal cancer.

In one embodiment said elevated urine concentration of valine is a urine concentration of more than 0.5 mmol/mL, more than 0.6 mmol/mL, more than 0.7 mmol/mL, more than 0.8 mmol/mL, more than 0.9 mmol/mL, more than 1.0 mmol/mL.

In one embodiment said elevated urine concentration of valine is between 0.5 mmol/mL and 1.3 mm/mL, between 0.6 mmol/mL and 1.3 mm/mL, between 0.7 mmol/mL and 1.3 mm/mL, between 0.8 mmol/mL and 1.3 mm/mL, between 0.9 mmol/mL and 1.3 mm/mL, between 1.0 mmol/mL and 1.3 mm/mL.

In one embodiment either or both of said metabolite profile and said reference metabolite profile are obtained using one or more methods selected from the group consisting of nuclear magnetic resonance spectroscopy, high performance liquid chromatography, thin layer chromatography, gas chromatography, electrochemical analysis, mass spectroscopy, refractive index spectroscopy, ultra-violet spectroscopy, fluorescent analysis, radiochemical analysis, near-infrared spectroscopy, capillary electrophoresis, and light scattering analysis.

In a further aspect the present invention relates to the use of a metabolite profile comprising at least the urine metabolite TMAO for assessing whether a subject has or is predisposed to developing metastatic colorectal cancer.

In one embodiment said metabolite profile further comprises the urine metabolite cis-aconitate.

In yet another aspect the present invention relates to a method for identifying and evaluating effectiveness of pharmaceutical agents and/or surgical treatments and/or physical treatments against metastatic colorectal cancer, said method comprising:
a) providing a first urine sample from a subject having metastatic colorectal cancer;
b) obtaining a first metabolite profile from said first urine sample, wherein said metabolic profile is obtained by measuring the concentration of at least the metabolite TMAO in said first urine sample to produce said metabolite profile for said subject;
c) providing a second urine sample from said subject to which after step b) one or more pharmaceutical agents have been administered and/or on which one or more physical or surgical treatments have been performed;
d) obtaining a metabolite profile from said second urine sample, wherein said metabolic profile is obtained by measuring the concentration of at the metabolite TMAO in said second urine sample to produce said second metabolite profile for said subject;
e) comparing said first and second metabolite profile obtained in steps b) and d)
f) assessing, based on said comparison in step e), whether the one or more pharmaceutical agents and/or treatments is/are effective against metastatic colorectal cancer.

In one embodiment of this method step b) and d) further comprise measuring the concentration of the metabolite cis-aconitate.

### BRIEF DESCRIPTION OF FIGURES

Figure 1: TMAO and cis-aconitate concentrations per study group, and TMAO concentrations per colorectal cancer stages (0-IV). PLY: Polyps; ALR: Adenoma Low Risk; AMR: Adenoma Medium Risk; AHR: Adenoma High Risk; CRC: colorectal cancer (stages 0-III); CRCm: colorectal cancer metastatic.
Figure 2: TMAO sensitivity, specificity and ROC. CRCe (stages 0-III) vs CRCm (stage IV).

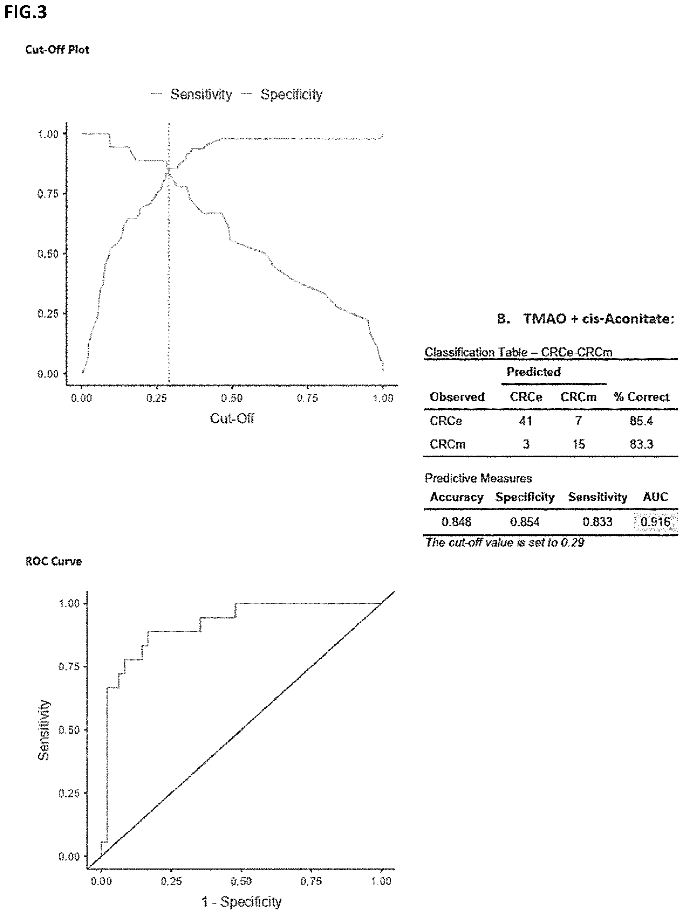

Figure 3: TMAO + cis-aconitate, sensitivity, specificity and ROC. CRCe (stages 0-III) vs CRCm (stage IV).

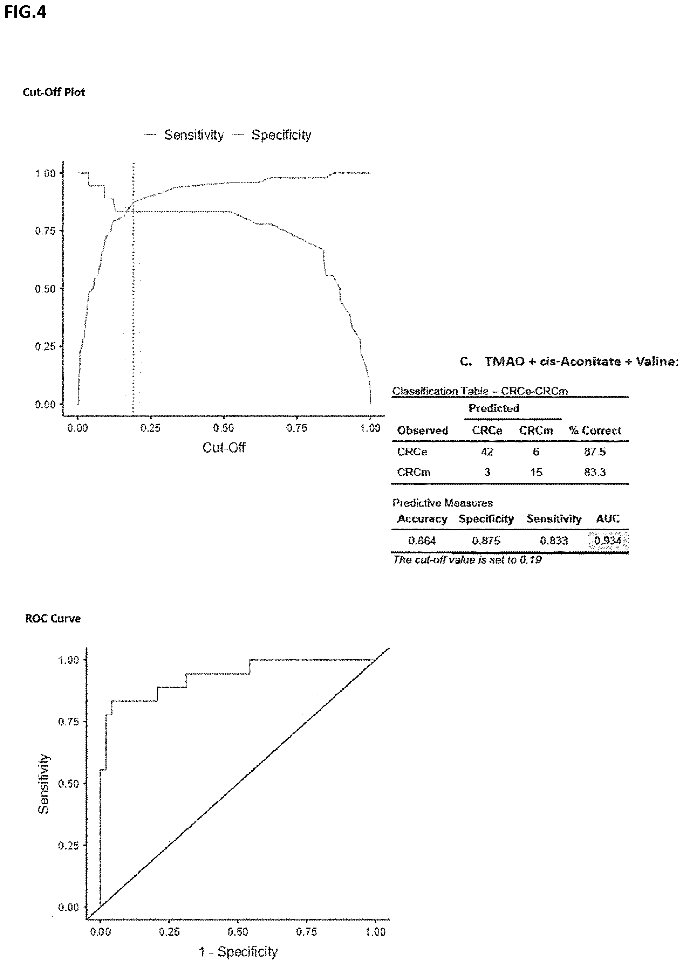

Figure 4: TMAO + cis-aconitate + valine, sensitivity, specificity and ROC. CRCe (stages 0-III) vs CRCm (stage IV)

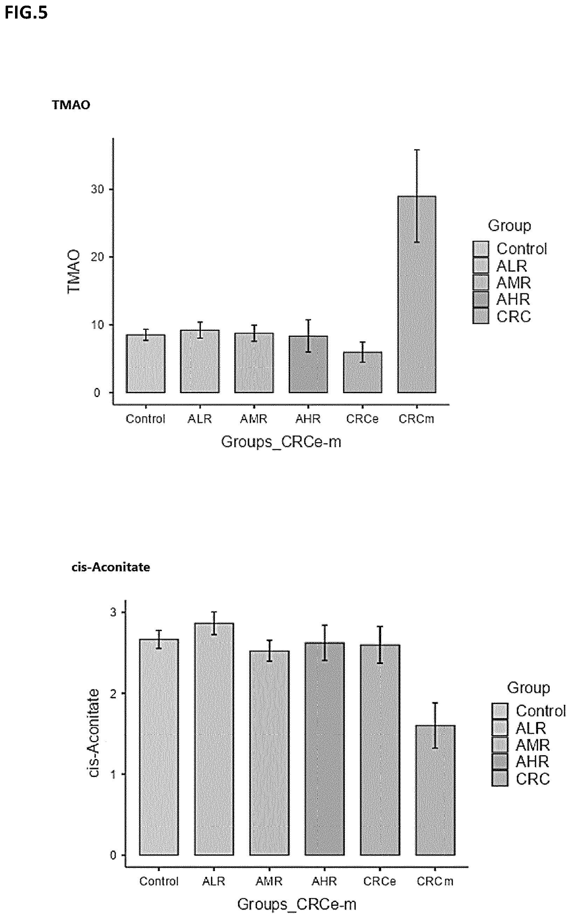

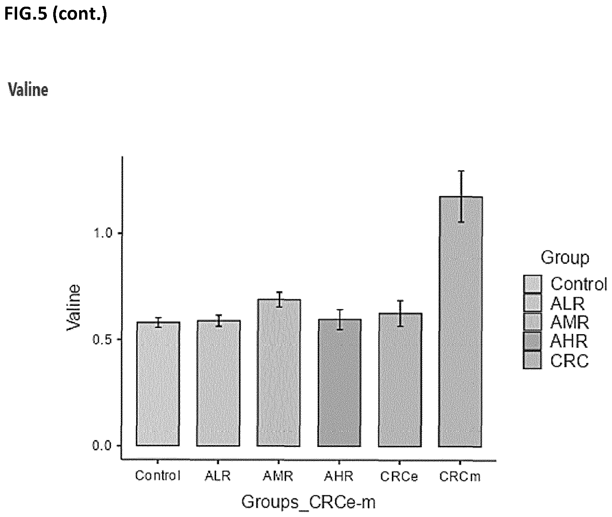

Figure 5: Bar plots of TMAO, cis-aconitate and valine, for all groups ALR: Adenoma Low Risk; AMR: Adenoma Medium Risk; AHR: Adenoma High Risk; CRCe: colorectal cancer (stages 0-III); CRCm: colorectal cancer metastatic (stage IV).

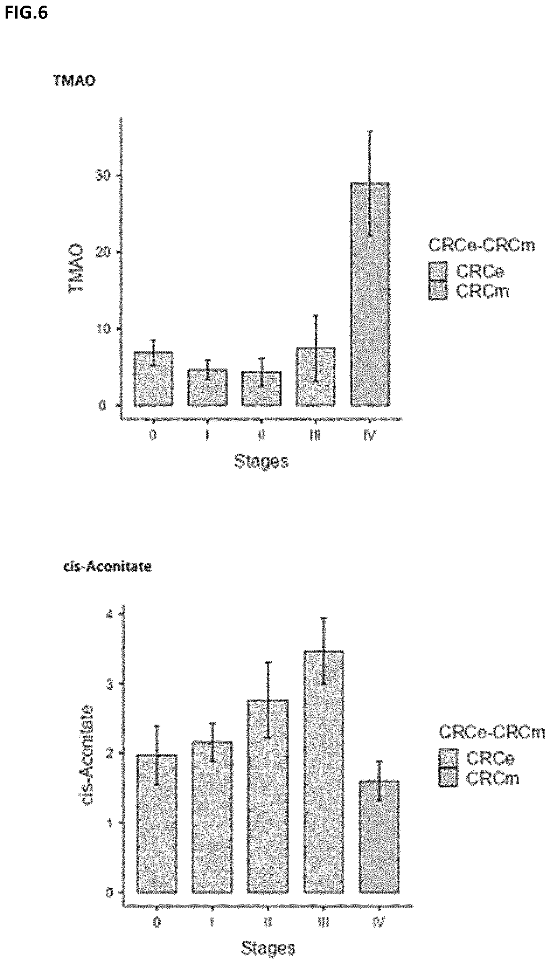

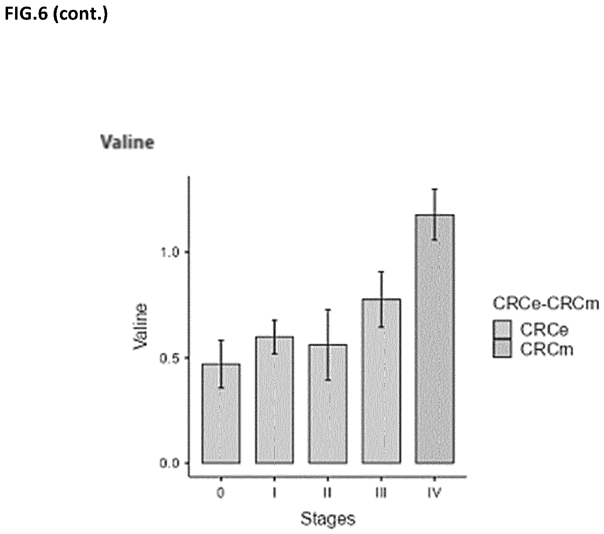

Figure 6: Bar plots of TMAO, cis-aconitate and valine, for early stages (CRCe: stages 0, I, II, and III) and metastatic stage (CRCm: stage IV).

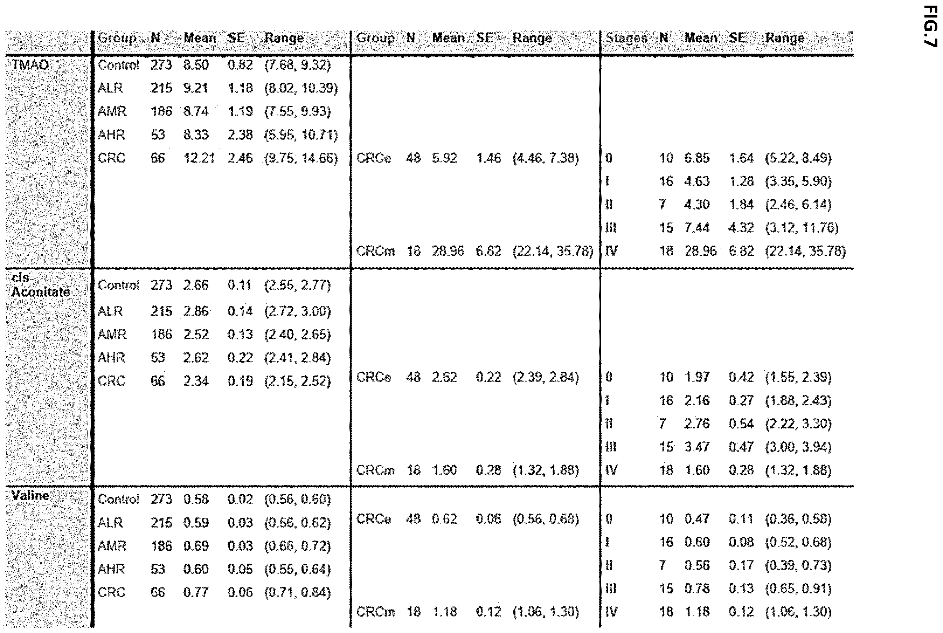

Figure 7: Ranges of TMAO, cis-aconitate and valine.

### DETAILED DESCRIPTION

The present invention may be understood more readily by reference to the following detailed description of the preferred embodiments of the invention, and to the examples included therein.

As mentioned above, there are two major problems in the management of colon cancer that are still not solved by the detection methods of the prior art. One is the discrimination of metastatic CRC from early CRC during initial diagnosis, and the other is the detection of CRC recurrence during postoperative surveillance. There is therefore a need for the development of a diagnostic method and device that increases the overall sensitivity, specificity, non-invasiveness and cost-effectiveness of the diagnosis of mCRC. Ultimately such a method and device can reduce unnecessary surgeries or biopsies in early, non-metastatic cases and earlier intervention in metastatic disease which will improve patient outcomes and lower the economic costs of treatment. The inventors have therefore set out to develop a method that can overcome these disadvantages.

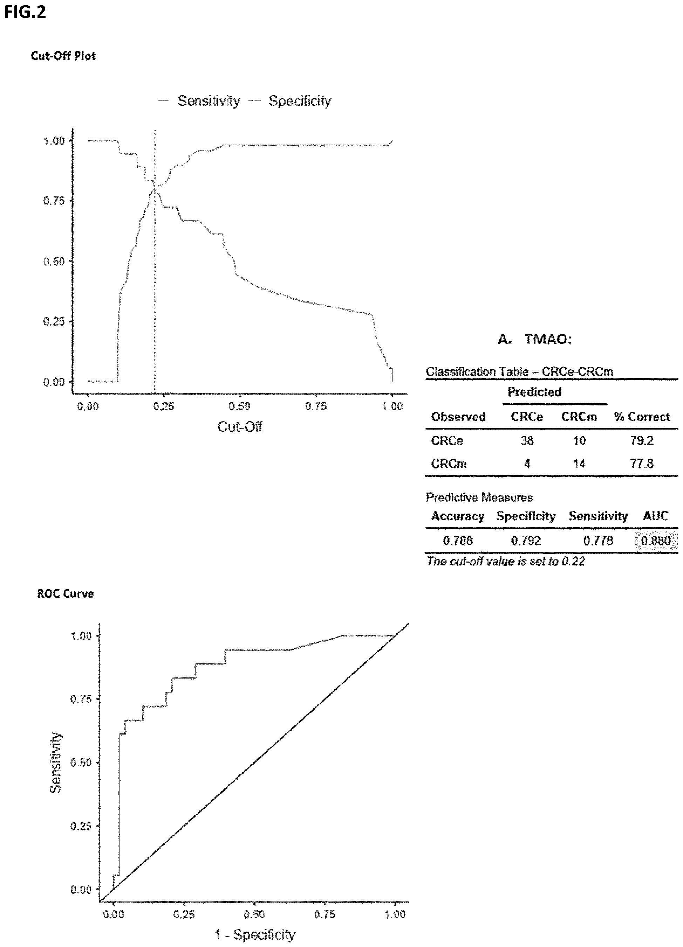

As described in more detail in Example 1, the inventors have assessed the presence of the metabolite TMAO in urine of different patients with different cancer types and have found that specifically in metastatic CRC, and only in that type, the TMAO levels are extremely elevated in the urine, as can be seen in figure 2A). They could furthermore show that the TMAO level increases with the CRC stages, the highest being in metastatic stage (IV) (figure 2C).

Furthermore, at the same time as TMAO levels were increased, cis-aconitate levels were found to be decreased in the metastatic stage of CRC (see figure 2B). The inventors have therefore identified two metabolic markers that are suitable to reliably predict in a urine sample whether a CRC patient is in the metastatic stage. The combination of TMAO and cis-aconitate as urine biomarkers allows the differentiation of stage IV (metastatic) CRC from earlier stages of the disease.

The present invention therefore relates in one aspect to a method for detecting whether a subject has or is predisposed to developing metastatic colorectal cancer, said method comprising:
(a) providing a urine sample from said subject;
(b) obtaining a metabolite profile from said urine sample, wherein said metabolic profile is obtained by measuring the concentration of at least the metabolite TMAO in said urine sample to produce said metabolite profile for said subject;
(c) comparing said metabolite profile with a reference metabolite profile, wherein said reference metabolite profile is determined from the concentration of corresponding metabolites in urine of individuals in a reference population; and
(d) assessing, based on said comparison in step (c), whether said subject has or is predisposed to developing metastatic colorectal cancer.

In one embodiment of the method of present invention an elevated urine concentration of TMAO is indicative of that the subject has or is predisposed to developing metastatic colorectal cancer.

In one embodiment said elevated urine concentration of TMAO is a urine concentration of more than 9 mmol/mL, preferably more than 9.5 mmol/mL, more preferably more than 10 mmol/mL.

In one embodiment, said elevated urine concentration can be up to 35 mmol/mL.

In one embodiment, said elevated urine concentration is between 9 mmol/mL to 35 mmol/mL.ln one embodiment of the method of present invention step b) furthermore comprises measuring the concentration of the metabolite cis-aconitate in said urine sample.

In one embodiment of the method of present invention the reduced urine concentration of cis-aconitate is indicative of that the subject has or is predisposed to developing metastatic colorectal cancer.

In one embodiment said reduced urine concentration of cis-aconitate is a urine concentration of less than 4 mmol/mL, preferably less than 3.5 mmol/mL, more preferably less than 3 mmol/mL, or less than 2.5 mmol/mL, most preferred less than 2.0 mmol/mL.

In one embodiment the urine concentration of cis-aconitate is between 4 mmol/mL and 1.3 mm/mL, 3.5 mmol/mL and 1.3 mm/mL, between 3 mmol/mL and 1.3 mm/mL, between 2.5 mmol/mL and 1.3 mm/mL, most preferred between 2.0 mmol/mL and 1.3 mm/mL.

In one embodiment the subject has colorectal cancer and wherein step d) comprises assessing whether the colorectal cancer is in the metastatic stage (stage VI).

In one embodiment said metabolic profile is obtained by further measuring the concentration of at least one other metabolite, wherein said other metabolite is valine.

In one embodiment the elevated urine concentration of valine is indicative of that the subject has metastatic colorectal cancer.

In one embodiment said elevated urine concentration of valine is a urine concentration of more than 0.5 mmol/mL, more than 0.6 mmol/mL, more than 0.7 mmol/mL, more than 0.8 mmol/mL, more than 0.9 mmol/mL, more than 1.0 mmol/mL.

In one embodiment said elevated urine concentration of valine is between 0.5 mmol/mL and 1.3 mm/mL, between 0.6 mmol/mL and 1.3 mm/mL, between 0.7 mmol/mL and 1.3 mm/mL, between 0.8 mmol/mL and 1.3 mm/mL, between 0.9 mmol/mL and 1.3 mm/mL, between 1.0 mmol/mL and 1.3 mm/mL.

In one embodiment either or both of said metabolite profile and said reference metabolite profile are obtained using one or more methods selected from the group consisting of nuclear magnetic resonance spectroscopy, high performance liquid chromatography, thin layer chromatography, gas chromatography, electrochemical analysis, mass spectroscopy, refractive index spectroscopy, ultra-violet spectroscopy, fluorescent analysis, radiochemical analysis, near-infrared spectroscopy, capillary electrophoresis, and light scattering analysis.

Representative examples of methods for measuring metabolites include Time-of-flight mass analyzers, Gas chromatography, capillary electrophoresis, Fourier transform ion cyclotron resonance (FT-ICR), LC-MS (moderately high-throughput), ultrahigh pressure liquid chromatography (UPLC), extractive electrospray ionization MS (EESI-MS), desorption electrospray atmospheric ionization MS (DESI-MS), direct analysis in real time MS (DART-MS), and matrix-assisted laser desorption/ionization MS (MALDI-MS).

In a further aspect the present invention relates to the use of a metabolite profile comprising at least the urine metabolite TMAO for assessing whether a subject has or is predisposed to developing metastatic colorectal cancer.

In one embodiment said metabolite profile further comprises the urine metabolite cis-aconitate.

The detection and quantitation of metabolites can also be useful for monitoring the efficacy and success of a treatment of the colorectal cancer in patients. A subject being treated with any pharmaceutical agent or being subjected to surgical or physical treatment against the metastatic colorectal cancer can be monitored during such treatment by simply taking a urine sample before and after, and also at any desired time intervals during the treatment. The urine samples can then be assessed with the method and/or device of present invention thus providing a simple, non-invasive way of monitoring the development of the CRC.

In yet another aspect the present invention relates to a method for identifying and evaluating effectiveness of pharmaceutical agents and/or surgical treatments and/or physical treatments against metastatic colorectal cancer, said method comprising:
a) providing a first urine sample from a subject having metastatic colorectal cancer;
b) obtaining a first metabolite profile from said first urine sample, wherein said metabolic profile is obtained by measuring the concentration of at least the metabolite TMAO in said first urine sample to produce said metabolite profile for said subject;
c) providing a second urine sample from said subject to which after step b) one or more pharmaceutical agents have been administered and/or on which one or more physical or surgical treatments have been performed;
d) obtaining a metabolite profile from said second urine sample, wherein said metabolic profile is obtained by measuring the concentration of at the metabolite TMAO in said second urine sample to produce said second metabolite profile for said subject;
e) comparing said first and second metabolite profile obtained in steps b) and d)
f) assessing, based on said comparison in step e), whether the one or more pharmaceutical agents and/or treatments is/are effective against metastatic colorectal cancer.

In one embodiment of this method step b) and d) further comprise measuring the concentration of the metabolite cis-aconitate.

In one embodiment of this method step b) and d) further comprise measuring the concentration of the metabolite valine.

In one embodiment of the method of the invention a decrease of at least 20% of the TMAO level in the second metabolite profile compared to in the first metabolite profile can be an indication of an effective treatment against metastatic colorectal cancer.

In one embodiment of the method of the invention an increase of at least 25% of the cis-aconitate level in the second metabolite profile compared to in the first metabolite profile can be an indication of an effective treatment against metastatic colorectal cancer.

In one embodiment of the method of the invention a decrease at least 20% of the valine level in the second metabolite profile compared to the first metabolite profile can be an indication of an effective treatment against metastatic colorectal cancer.

As used herein the term "colorectal cancer" or "CRC" refers to a malignant epithelial tumour arising from the colonic or rectal mucosa. TNM staging is defined as follows for colorectal cancer: Stage I: Tumour invades muscularis propria, but no spread to nearby lymph nodes; Stage II: Tumour spreads into the subserosa and/or perirectal tissues with up to 3 regional lymph nodes, OR directly invades adjacent tissue without lymph node involvement; Stage III: Any depth of tumour invasion with four or more positive lymph nodes; no distant metastases; Stage IV: Any depth of tumour invasion; any number positive lymph nodes, distant metastases.

As used herein the term "metastatic colorectal cancer" refers to stage IV of the colorectal cancer as described above.

The use of the word "a" or "an" may mean "one," but it is also consistent with the meaning of "one or more," "at least one," and "one or more than one". The use of the term "another" may also refer to one or more. The use of the term "or" in the claims is used to mean "and/or" unless explicitly indicated to refer to alternatives only or the alternatives are mutually exclusive.

As used herein, words of approximation such as, without limitation, "about", "around", "approximately" refers to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skilled in the art recognize the modified feature as still having the required characteristics and capabilities of the unmodified feature. In general, but subject to the preceding discussion, a numerical value herein that is modified by a word of approximation such as "about" may vary from the stated value by ±1, 2, 3, 4, 5, 6, 7, 8, 9, or 10%. Accordingly, the term "about" may mean the indicated value ± 5% of its value, preferably the indicated value ± 2% of its value, most preferably the term "about" means exactly the indicated value (± 0%).

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The term "comprises" also encompasses and expressly discloses the terms "consists of" and "consists essentially of". As used herein, the phrase "consisting essentially of" limits the scope of a claim to the specified materials or steps and those that do not materially affect the basic and novel characteristic(s) of the claimed invention. As used herein, the phrase "consisting of" excludes any element, step, or ingredient not specified in the claim except for, e.g., impurities ordinarily associated with the element or limitation.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

The terms "treatment" and "therapy", as used in the present application, refer to a set of hygienic, pharmacological, surgical and/or physical means used with the intent to cure and/or alleviate a disease and/or symptoms with the goal of remediating the health problem. The terms "treatment" and "therapy" include preventive and curative methods, since both are directed to the maintenance and/or reestablishment of the health of an individual or animal. Regardless of the origin of the symptoms, disease and disability, the administration of a suitable medicament to alleviate and/or cure a health problem should be interpreted as a form of treatment or therapy within the context of this application. Preferably, the term "treatment" refers to the application or administration of a pharmaceutical composition to a subject who has a disease or condition characterized by ER stress, with the purpose to cure, heal, alleviate, relieve, alter, remedy, ameliorate, improve, or affect the disorder, the symptom of the disease, or the predisposition toward a disease.

The terms "individual", "patient" or "subject" are used interchangeably in the present application and are not meant to be limiting in any way. The "individual", "patient" or "subject" can be of any age, sex and physical condition.

### Examples:

### Example 1: Urinary Metabolomics with 25 compounds

Urine samples were collected in fasting condition and kept in a fridge at 4°C until same-day transport to the biobank, where they were aliquoted and stored at -80°C. Participants belong to two cohorts from Hospital Universitari Sant Joan de Reus, the UrinCCR cohort (ceim ref. 074/2018) of metastatic CRC patients and the EarlyCRC cohort (ceim ref. 114/2019) of participants in the CRC screening program. The EarlyCRC participants' health status was confirmed with a colonoscopy. All participants were anonymized.

Urine samples were thawed at room temperature before 540 µL of urine was mixed with 60 µL of buffer (KH2PO4, 1.5 M, pH 7.4 made up in H2O) containing 5.8 mM TSP and 2mM NaN3. Following centrifugation (12000 g, 4°C, 5 min) to remove solids, 550 µL of sample is transferred into 5 mm SampleJet NMR tubes and immediately loaded onto a refrigerated SampleJet robot (Bruker Biospin, Germany) and maintained at 4°C until analysis. High-resolution 1H-NMR spectroscopy data was acquired on a Bruker 600 MHz Spectrometer.

Urine analysis with 1H-NMR quantified up to 27 metabolites. Two metabolites were not included in the analysis because one had over 60% of missing values, and the other was found with principal components analysis (PCA) to be an outlier. Therefore, all analyses have been carried out with 25 metabolites.

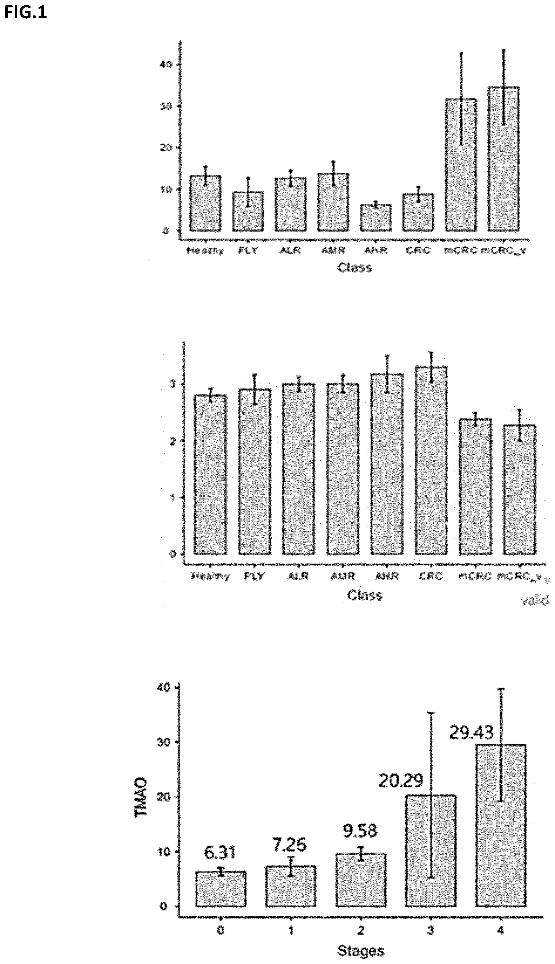

From the univariate and multivariate statistical analysis, 8 metabolites were found relevant, however they were reduced to three with a LASSO selection: TMAO, cis-aconitate, and valine. Noteworthy, TMAO is specific of colorectal cancer staging (Fig 1C). The area under the curve (AUC) of the CRC progression model is 0.890, which means we have a very good model to explain the differences between early and metastatic CRC groups. The model ROC curve has been created with a random forest algorithm for biomarkers 1 and 2. The permutation test of the model has an observed statistic at p = 0.0126, therefore, being a significant model (<0.05). The prediction test was further evaluated with a validation set of metastatic cancer patients, obtaining a significant model with a similar AUC of 0.880 (Fig 2) and p = 0.0131. The test has an accuracy of 84.8% (Fig 2), a sensitivity of 83.3% and a specificity of 85.4%, with a positive predictive value (PPV) of 83.3% and a negative predictive value (NPV) of 85.4%. Outperforming CEA blood test (47% sensitivity and 80% specificity).

### References

(1) Int. Agency for Research on Cancer. Global Cancer Observatory: Cancer Today. 2021. https://gco.iarc.fr/today/home.
(2) Denlinger CS, Sanft T, Moslehi JJ, Overholser L, Armenian S, Baker KS, Broderick G, Demark-Wahnefried W, Friedman DL, Goldman M, Henry NL, Hill-Kayser C, Hudson M, Khakpour N, Koura D, McDonough AL, Melisko M, Mooney K, Moore HCF, Moryl N, O'Connor T, Paskett ED, Patel C, Peterson L, Pirl W, Rodriguez MA, Ruddy KJ, Shockney L, Smith S, Syrjala KL, Tevaarwerk A, Zee P, McMillian NR, Freedman-Cass DA. NCCN Guidelines Insights: Survivorship, Version 2.2020. J Natl Compr Canc Netw. 2020 Aug;18(8):1016-1023. doi: 10.6004/jnccn.2020.0037. PMID: 32755975; PMCID: PMC7785060.
(3) Locker GY, Hamilton S, Harris J, Jessup JM, Kemeny N, Macdonald JS, Somerfield MR, Hayes DF, Bast RC Jr; ASCO. ASCO 2006 update of recommendations for the use of tumor markers in gastrointestinal cancer. J Clin Oncol. 2006 Nov 20;24(33):5313-27. doi: 10.1200/JCO.2006.08.2644. Epub 2006 Oct 23. PMID: 17060676.
(4) Gao Y, Wang J, Zhou Y, Sheng S, Qian SY, Huo X. Evaluation of Serum CEA, CA19-9, CA72-4, CA125 and Ferritin as Diagnostic Markers and Factors of Clinical Parameters for Colorectal Cancer. Sci Rep. 2018 Feb 9;8(1):2732. doi: 10.1038/s41598-018-21048-y. PMID: 29426902; PMCID: PMC5807317.
(5) Garcia-Figueiras R, Baleato-González S, Padhani AR, Luna-Alcal6 A, Marhuenda A, Vilanova JC, Osorio-Vázquez I, Martinez-de-Alegria A, Gómez-Caamaño A. Advanced Imaging Techniques in Evaluation of Colorectal Cancer. RadioGraphics 2018 May-Jun;38(3) :740-765. doi: 10.1148/rg.2018170044. Epub 2018 Apr 20. PMID: 29676964.
(6) Tan B, Qiu Y, Zou X, Chen T, Xie G, Cheng Y, Dong T, Zhao L, Feng B, Hu X, Xu LX, Zhao A, Zhang M, Cai G, Cai S, Zhou Z, Zheng M, Zhang Y, Jia W. Metabonomics identifies serum metabolite markers of colorectal cancer. J Proteome Res. 2013 Jun 7;12(6):3000-9. doi: 10.1021/pr400337b. Epub 2013 May 29. PMID: 23675754; PMCID: PMC5902797.
(7) Xu, R, Wang Q, Li L. A genome-wide systems analysis reveals strong link between colorectal cancer and trimethylamine N-oxide (TMAO), a gut microbial metabolite of dietary meat and fat. BMC Genomics. 201516 Suppl 7(Suppl 7):S4. doi: 10.1186/1471-2164-16-S7-S4. Epub 2015 Jun 11. PMID: 26100814; PMCID: PMC4474417.
(8) Liu X, Liu H, Yuan C, Zhang Y, Wang W, Hu S, Liu L, Wang Y. Preoperative serum TMAO level is a new prognostic marker for colorectal cancer. Biomark Med. 2017 May;11(5):443-447. doi: 10.2217/bmm-2016-0262. PMID: 28621609.
(9) Cheng Y, Xie G, Chen T, Qiu Y, Zou X, Zheng M, Tan B, Feng B, Dong T, He P, Zhao L, Zhao A, Xu LX, Zhang Y, Jia W. Distinct urinary metabolic profile of human colorectal cancer. J Proteome Res. 2012 Feb 3;11(2):1354-63. doi: 10.1021/pr201001a. Epub 2011 Dec 28. PMID: 22148915.

## Claims

1. A method for detecting whether a subject has or is predisposed to developing metastatic colorectal cancer, said method comprising:
(a) providing a urine sample from said subject;
(b) obtaining a metabolite profile from said urine sample, wherein said metabolic profile is obtained by measuring the concentration of at least the metabolite TMAO in said urine sample to produce said metabolite profile for said subject;
(c) comparing said metabolite profile with a reference metabolite profile, wherein said reference metabolite profile is determined from the concentration of corresponding metabolites in urine of individuals in a reference population; and
(d) assessing, based on said comparison in step (c), whether said subject has or is predisposed to developing metastatic colorectal cancer.

2. The method according to claim 1, wherein an elevated urine concentration of TMAO is indicative of that the subject has or is predisposed to developing metastatic colorectal cancer.

3. The method of claim 2, wherein the elevated urine concentration of TMAO is a urine concentration of more than 9 mmol/mL.

4. The method of any one of the preceding claims, wherein step b) furthermore comprises measuring the concentration of the metabolite cis-aconitate in said urine sample.

5. The method of claim 4, wherein the reduced urine concentration of cis-aconitate is indicative of that the subject has or is predisposed to developing metastatic colorectal cancer.

6. The method of claim 5, wherein the reduced urine concentration of cis-aconitate is a urine concentration of less than 4 mmol/mL.

7. The method according to any one of the preceding claims, wherein said metabolic profile is obtained by further measuring the concentration of at least one other metabolite, wherein said other metabolite is valine.

8. The method of claim 7, wherein the elevated urine concentration of valine is indicative of that the subject has metastatic colorectal cancer.

9. The method of claim 8, wherein the elevated urine concentration of valine is a urine concentration of more than 0.5 mmol/mL.

10. The method according to any one of claims 1 to 9, wherein either or both of said metabolite profile and said reference metabolite profile are obtained using one or more methods selected from the group consisting of nuclear magnetic resonance spectroscopy, high performance liquid chromatography, thin layer chromatography, gas chromatography, electrochemical analysis, mass spectroscopy, refractive index spectroscopy, ultra-violet spectroscopy, fluorescent analysis, radiochemical analysis, near-infrared spectroscopy, gas chromatography and light scattering analysis.

11. Use of a metabolite profile comprising at least the urine metabolite TMAO for assessing whether a subject has or is predisposed to developing metastatic colorectal cancer.

12. Use of claim 11, further comprising the urine metabolite cis-aconitate and/or the metabolite valine.

13. A method for identifying and evaluating effectiveness of pharmaceutical agents and/or surgical treatments and/or physical treatments against metastatic colorectal cancer, said method comprising:
a) providing a first urine sample from a subject having metastatic colorectal cancer;
b) obtaining a first metabolite profile from said first urine sample, wherein said metabolic profile is obtained by measuring the concentration of at least the metabolite TMAO in said first urine sample to produce said metabolite profile for said subject;
c) providing a second urine sample from said subject to which after step b) one or more pharmaceutical agents have been administered and/or on which one or more physical or surgical treatments have been performed;
d) obtaining a metabolite profile from said second urine sample, wherein said metabolic profile is obtained by measuring the concentration of at least the metabolite TMAO in said second urine sample to produce said second metabolite profile for said subject;
e) comparing said first and second metabolite profile obtained in steps b) and d)
f) assessing, based on said comparison in step e), whether the one or more pharmaceutical agents and/or treatments is/are effective against colorectal cancer.

14. The method of claim 13, wherein step b) and d) further comprise measuring the concentration of the metabolite cis-aconitate.

15. The method of claims 13 or 14, wherein step b) and d) further comprise measuring the concentration of the metabolite valine.
